# EUROPÄISCHE PATENTSCHRIFT

(11) **EP 2 158 193 B1**
(45) Veröffentlichungstag und Bekanntmachung des Hinweises auf die Patenterteilung: **08.08.2012**
(21) Anmeldenummer: 08760040.9
(22) Anmeldetag: 27.05.2008
(51) Int. Cl.: C07D 333/08

(54) **VERFAHREN ZUR HERSTELLUNG ALKYLSUBSTITUIERTER THIOPHENE**
METHOD FOR THE PRODUCTION OF ALKYL-SUBSTITUTED THIOPHENES
PROCÉDÉ DE PRODUCTION DE COMPOSÉS THIOPHÈNES ALCOYLÉS

(30) Priorität: 09.06.2007 DE 102007026763
(43) Veröffentlichungstag der Anmeldung: 03.03.2010
(73) Patentinhaber: Saltigo GmbH, 40764 Langenfeld (DE)
(72) Erfinder: HIERONYMI, Antje, 50733 Köln (DE); RODEFELD, Lars, 51375 Leverkusen (DE)
(74) Vertreter: Pettrich, Klaus-Günter
(86) Internationale Anmeldenummer: PCT/EP2008/056442
(87) Internationale Veröffentlichungsnummer: WO 2008/151920

(56) Entgegenhaltungen:
- US-A1- 2006 155 134
- TAMAO K ET AL: "Nickel-phosphine complex-catalysed Grignard coupling - II" TETRAHEDRON, ELSEVIER SCIENCE PUBLISHERS, AMSTERDAM, NL, Bd. 38, Nr. 22, 1. Januar 1982 (1982-01-01), Seiten 3347-3354, XP002251876 ISSN: 0040-4020

## Beschreibung

Die vorliegende Erfindung betrifft ein Verfahren zur Herstellung von substituierten Thiophenen. Die Verbesserung gegenüber den bislang bekannten Verfahren besteht in der Verbesserung der Selektivität.

Substituierte Heteroaromaten sind wertvolle Intermediate in der Elektronikindustrie. Sie werden beispielsweise in den Anwendungsbereichen Beleuchtung, Solarzellen und Displays eingesetzt und schaffen neue Möglichkeiten hinsichtlich Formgebung, Verarbeitung und Energieeffizienz. Im Vergleich zu etablierten Produkten bieten sie zudem wirtschaftliche Anreize, da ihre Herstellung wesentlich weniger aufwändig ist. Insbesondere Thiophene können aufgrund ihrer Eigenschaft, nach Polymerisation konjugierte Doppelbindungen aufzubauen, in unterschiedlichen Strukturelementen der genannten Anwendungen eingesetzt werden.

Das erste Verfahren zur Herstellung von substituierten Heteroaromaten ausgehend von halogenierten Aromaten und Grignard-Reagenzien wurde von Kumada et al. entwickelt (Tetrahedron 1982, 38, 3347-3354). Hier wurden diverse Alkyl- und Arylreste in verschiedenen Positionen von Thiophenen, Pyridinen und Quinolinen eingeführt. Auch Mehrfachalkylierungen wurden durchgeführt, indem mehrfach halogenierte Heteroaromaten mit Grignard-Verbindungen unter Nickelkatalyse umgesetzt wurden. Als Lösemittel für diese Reaktionen wurde Diethylether eingesetzt. Nachteilig ist neben der Verwendung des leichtsiedenden und stark zur Peroxidbildung neigenden Diethylethers, dass bei diesem Verfahren signifikante Mengen der Homokupplungsprodukte (z.B. Dithiophen bei der Reaktion von 3-Bromthiophen mit n-Hexylmagnesiumbromid) gebildet werden, welche die weitere Reinigung erschweren.

Weiterhin wird in Synth. Commun. 1986, 16, 689-696 von Zimmer *et al.* die Kumada-Kupplung von Bromthiophen mit Alkylmagnesiumhalogeniden beschrieben. Hier wird unter Anderem die Kupplung von 3-Bromthiophen mit n-Hexylmagnesiumbromid in Diethylether durchgeführt, die Autoren erhalten eine isolierte Ausbeute von 71 %.

Aus der US 2006/0155134 ist bekannt, dass Kumada-Kupplungen von 3-Halogenthiophenen mit Alkyl- und Arylmagnesiumhalogeniden in Methyltetrahydrofuranen durchgeführt werden können. Die Durchführung in diesem Lösemittel reduziert das Nebenproduktspektrum gegenüber den Verfahren, die z.B. Diethylether oder Tetrahydrofuran einsetzen. Nachteilig an diesem Verfahren ist, dass isomere, verzweigte Nebenkomponenten in der Größenordnung um 0,5 % anfallen.

Die US 2006/0155134 beschreibt, dass die Verwendung von tert-Butylmethylether bei der beschriebenen Kumada-Kupplung eines Alkylrestes an 3-Halogenthiophene ein schlechtes Ergebnis hinsichtlich des Nebenkomponentenspektrums, insbesondere in der Bildung von Dithiophenen liefert.

Aufgabe war es, ein verbessertes Verfahren zur Herstellung von substituierten Thiophenen zu entwickeln, welches zu weniger Nebenkomponenten führt.

Gegenstand der Erfindung ist daher ein Verfahren zur Herstellung von substituierten Thiophenen der allgemeinen Formel (I) 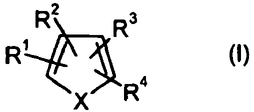 wobei
- X: Schwefel
und
- R⁴: C₁-C₂₀-Alkyl,
- R¹, R², R³: Wasserstoff, Halogen, C₁-C₂₀-Alkyl, C₅-C₆-Aryl oder Heteroaryl ist, durch Umsetzung

### A) eines halogenierten Heteroaromaten der allgemeinen Formel (II)

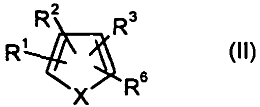

wobei
- X: die für Formel (I) angegebene Bedeutung und
- R⁶: Brom, Iod oder Chlor ist und
- R¹, R² und R³: die für Formel (I) angegebene Bedeutung haben,
mit einem Grignard-Reagenz der allgemeinen Formel (III)

R⁴MgHal (III)

wobei
- R⁴: die für Formel (I) angegebene Bedeutung hat und
- Hal: Brom, Iod oder Chlor ist oder

### B) Umsetzung des halogenierten Heteroaromaten der Formel (II) mit Magnesium zunächst zu einer Grignard-Verbindung der allgemeinen Formel (IIIa)

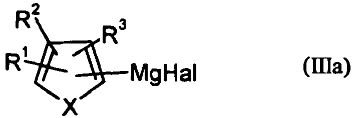

wobei
- Hal: Brom, Iod oder Chlor ist und
- X und R¹, R² und R³: die für Formel (I) angegebene Bedeutung haben,
und weiterer Umsetzung mit einer halogenierten Verbindung der allgemeinen Formel (IV)

R⁴Hal (IV)

wobei
- R⁴: die für Formel (I) angegebene Bedeutung hat und
- Hal: Brom, Iod oder Chlor ist,
wobei die Umsetzungen A) oder B) jeweils in Gegenwart eines Ni- oder Pd-Katalysators durchgeführt werden, dadurch gekennzeichnet, dass das Verfahren in Gegenwart von Cycloalkylalkylether als Lösungsmittel und gegebenenfalls einem oder mehreren weiteren Lösungsmitteln durchgeführt wird.

Der Cycloalkylalkylether ist bevorzugt Cyclopentylmethylether.

Als Katalysator wird eine Nickel- oder eine Palladium-Verbindung, bevorzugt ein Nickel(II)chlorid, besonders bevorzugt [1,3-Bis-(Diphenylphosphino)-propan]-Nickel(II)-chlorid, in einer Stöchiometrie von 0,001-20 mol-%, bevorzugt 0,05-1,5 mol%, besonders bevorzugt 0,1-0,5 mol% eingesetzt.

Magnesium wird in einer Stöchiometrie von 80-150 mol%, bevorzugt 100-140 mol%, besonders bevorzugt 105-130 mol-% eingesetzt.

Das das Grignard-Reagenz bildende Alkylhalogenid der allgemeinen Formel (IV) weist eine Stöchiometrie von 80-150 mol%, bevorzugt 100-140 mol%, besonders bevorzugt 105-130 mol% auf.

Die Lösung des Grignard-Reagenz der allgemeinen Formel (III) sollte eine Konzentration von 0,1-4 mol/L, bevorzugt 0,5-4 mol/L, besonders bevorzugt 1,5-3,5 mol/L haben.

Im Verfahren gemäß dieser Erfindung wird, wie bei Grignard-Reaktionen üblich, unter inerten Bedingungen gearbeitet. So wird die für die Reaktion benutzte Apparatur gegebenenfalls ausgeheizt und mit Stickstoff oder Argon befüllt. Auch bei der Zugabe von Rohstoffen sowie der Entnahme von Proben wird die inerte Atmosphäre aufrechterhalten, wobei insbesondere auf Wasserfreiheit zu achten ist.

Im vorliegenden Verfahren wird Magnesium bevorzugt in Cyclopentylmethylether in einer Syntheseapparatur vorgelegt. Dabei kann das Magnesium beispielsweise in Form von Stücken, Spänen, Granulat oder Pulver oder Mischungen dieser Einsatzformen eingesetzt werden. Die Konzentration von Magnesium in Cyclopentylmethylether beträgt erfindungsgemäß 0,1-4 mol/L, bevorzugt 0,5-4 mol/L, besonders bevorzugt 1,5-3,5 mol/L. Es ist auch möglich, Magnesium in einer Mischung aus Cyclopentylmethylether mit einem anderen Lösemittel vorzulegen, beispielsweise aber nicht einschränkend seien genannt: Toluol, aber auch Ether wie Tetrahydrofuran, Methyltetrahydrofuran, tert-Butylmethylether oder Diethylether oder Mischungen der vorgenannten Lösemittel.

Im Anschluss wird ein Alkyl- oder Arylhalogenid der allgemeinen Formel (IV) oder (II) wie oben beschrieben mit dem Magnesium zu einem Grignard-Reagenz der Formel (III) oder (IIIa), umgesetzt. Dabei ist Alkyl eine Alkylgruppe mit C₁-C₂₀, bevorzugt C₄-C₂₀, besonders bevorzugt C₄-C₁₀ besonders bevorzugt werden einfach halogenierte, alkylierte Thiophene oder zweifach halogenierte Dithiophene eingesetzt. In beiden Fällen sind die Halogene bevorzugt Chlor, Brom oder Iod, besonders bevorzugt Brom.

Das Alkyl- oder Arylhalogenid kann pur oder verdünnt in einem Lösemittel zugegeben werden. Lösemittel sind beispielsweise Cyclopentylmethylether, Tetrahydrofuran, Methyltetrahydrofuran, tert-Butylmethylether, Diethylether, Toluol oder Mischungen der vorgenannten Lösungsmittel. Die Molarität des Grignard-Reagenz beträgt bevorzugt 0,5-4 mol/L, besonders bevorzugt 1,5-3,5 mol/L.

Für im erfindungsgemäßen Verfahren eingesetzten Grignard-Reagenzien gelten die allgemeinen Eigenschaften von Grignard-Reagenzien. Sie sind luft- und wasserempfindlich und sollten somit unter Stickstoff- oder Edelgasatmosphäre gehandhabt und gelagert werden. Die Lagerung ist im Allgemeinen möglichst kurz zu halten, d.h. die Grignard-Reagenzien sollten nach Möglichkeit frisch hergestellt werden. Des Weiteren ist die Reaktion von Grignard-Reagenzien mit Wasser stark exotherm und Wasserstoff wird freigesetzt. Aus diesem Grunde sind Behälter von Grignard-Reagenzien vor Wassereintritt zu schützen.

Die im erfindungsgemäßen Verfahren eingesetzten Grignard-Reagenzien der Formel (III) oder (IIIa) gehen im Verlauf der Reaktion eine Übergangsmetallkatalysierte Reaktion mit einem Halogenid der allgemeinen Formel (IV) oder einem halogenierten Heteroaromat der allgemeinen Formel (II) mit den Resten wie oben beschrieben, ein. Dabei wird das Halogen des Halogenids durch die entsprechende Gruppe des Grignard-Reagenz ausgetauscht. Bevorzugt ist dabei die Umsetzung A), in der R⁶ gegen R⁴ aus dem Grignard-Reagenz ausgetauscht wird.

Der Katalysator für das in dieser Erfindung beschriebene Verfahren kann Nickel oder Palladium in der Oxidationsstufe 0 oder II sein, also Ni(II), Ni(0), Pd(II) oder Pd(0). Bevorzugt für dieses Verfahren sind [1,3-Bis(diphenylphosphino)propan]-Nickel(II)-chlorid und Nickeldichlorid sowie Mischungen daraus oder Suspensionen aus einem der Katalysatoren oder Suspensionen von Mischungen dieser Katalysatoren. Es könnten auch weitere bevorzugte Nickelkatalysatoren aus der Gruppe: Nickel(II)acetat, [1,2-Bis(diphenylphosphino)ethan]-Nickel(II)-chlorid, Hexaammin-nickel(β)chlorid, Nickel(II)acetylacetonat, oder Komplexe von Nickel(II)acetylacetonat mit Tri*tert*-butylphosphin, 1,3-Bis(2,4,6-trimethylphenyl)imidazolidiniumchlorid, 1,3-Bis(2,6-diisopropylphenyl)imidazolidiniumchlorid, 1,3-Diadamantylimidazoliumchlorid, Triadamantylphosphin, 1,3,4-triphenyl-4,5-dihydro-1H-1,2,4-triazol-5-yliden, 1,1'-Bis( diphenylphosphino)-ferrocen, 2-(Dicylcohexylphosphino)biphenyl, sowie Kombinationen aus allen oben genannten Katalysatoren sowie deren Suspensionen eingesetzt werden. Besonders bevorzugt im Verfahren der vorliegenden Erfindung ist die Verwendung von [1,3-Bis(diphenylphosphino)propan]-Nickel(II)-chlorid in Form einer Suspension oder als reines Einsatzmaterial.

In der vorliegenden Erfindung wird z.B. [1,3-Bis(diphenylphosphino)propan]-Nickel(II)-chlorid als Feststoff oder Suspension zu der wie oben beschrieben hergestellten Grignard-Lösung zugefügt. Es ist auch möglich, die Grignard-Lösung zu einer Suspension des Katalysators in Cyclopentylmethylether oder einem anderen geeigneten Lösemittel wie Tetrahydrofuran, Methyltetrahydrofuran, tert-Butylmethylether, Dioxan, Toluol oder Diethylether oder Mischungen aus allen oben genannten Lösemitteln zu geben. Auch kann in dieser Suspension bereits der halogenierte Heteroaromat gelöst sein. In der besonders bevorzugten Durchführung allerdings wird [1,3-Bis(diphenylphosphino)propan]-Nickel(II)-chlorid ohne weiteres Lösemittel zu dem Grignard-Reagenz zudosiert. Die Menge des benötigten Katalysators liegt zwischen 0,001-20 mol-%, bevorzugt 0,05-1,5 mol-%, besonders bevorzugt 0,1-0,5 mol-%. Die Konzentration der möglichen Katalysatorsuspension liegt zwischen 20 und 80 Gew.% im geeigneten Lösemittel.

Die Reaktion des Grignard-Reagenz mit dem Halogenid R⁴Hal der oben beschriebenen allgemeinen Formel (IV) wird durch das Zugeben des Halogenids zu der wie oben beschrieben hergestellten Mischung aus Grignard-Reagenz mit Katalysator initiiert. Sie kann ebenso durch die Zugabe des Grignard-Reagenz zu einer Mischung aus dem Halogenid und Katalysator in Cyclopentylmethylether oder einer Mischung aus Cyclopentylmethylether und einem weiteren Lösemittel hervorgerufen werden. Bevorzugt wird bei der vorliegenden Durchführungsweise das Zutropfen eines halogenierten Heteroaromaten, zu einer Mischung aus dem entsprechenden Grignard-Reagenz und [1,3-Bis(diphenylphosphino)propan]-Nickel(II)-chlorid in Cyclopentylmethylether.

X ist in Formel (I), **(II)** und (IIIa), Schwefel, d.h., das eingesetzte Halogenid der Formel **(II)** ist bevorzugt ein substituiertes oder unsubstituiertes Halogenthiophen, bevorzugt ein Chlorthiophen, Bromthiophen oder Iodthiophen, besonders bevorzugt Bromthiophen, ganz besonders bevorzugt 3-Bromthiophen. Es kann pur oder in Lösemittel, beispielsweise Toluol, Cyclopentylmethylether, tert-Butylmethylether, Diethylether, Tetrahydrofuran oder Methyltetrahydrofuran eingesetzt werden.

Das Verhältnis von Grignard-Reagenz zu halogeniertem Heteroaromat kann 80-150 mol-% betragen, bevorzugt werden 100-140 mol% eingesetzt, besonders bevorzugt 105-130 mol-%.

R¹, R² und R³ sind bevorzugt Wasserstoff und R⁴ ist bevorzugt eine C₁-C₁₂Alkylgruppe. Besonders bevorzugt ist R⁴ eine C₆-C₁₀- oder C₁₂-Alkylgruppe(Hexyl-, Decyl-, Dodecylgruppe). Es werden somit bevorzugt mit einer Hexyl-, Decyl-, Dodecylgruppe substituierte Thiophene, insbesondere in der 3-Position substituierte Thiophene, erfindungsgemäß hergestellt. 3-Hexylthiophen ist dabei ganz besonders bevorzugt.

Die Reaktionstemperatur wird nach Initiierung der Reaktion durch die Zutropfgeschwindigkeit und die Gegenkühlung gesteuert. Die Reaktionstemperatur sollte zwischen -30 und 106°C (Siedepunkt von Cyclopentylmethylether) liegen, bevorzugt sind Temperaturen zwischen 10 und 90°C, besonders bevorzugt liegen die Temperaturschwankungen zwischen 15 und 70°C.

Das Verfahren bietet eine Möglichkeit, halogenierte Heteroaromaten in nahezu vollständigem Umsatz in substituierte Heteroaromaten zu überführen, ohne dabei isomere Nebenkomponenten, wie z.B. bei der Reaktion von 3-Bromthiophen mit Hexylmagnesiumbromid das Isomer 3-(1-Methylpentyl)thiophen, in nennenswerten Mengen zu erhalten. Bei dieser Reaktion werden in überraschender Weise bei der Verwendung von Cyclopentylmethylether oder Mischungen aus Cyclopentylmethylether mit anderen Lösemitteln nur sehr geringe Mengen Dithiophene gebildet. Darüber hinaus bietet die Verwendung von Cyclopentylmethylether gegenüber der Verwendung von Methyltetrahydrofuranen den Vorteil, dass in der Rohmischung ein erheblich kleinerer Anteil der unerwünschten Nebenkomponente 3-(1-Methylpentyl)thiophen gefunden wird.

So sind ausgehend von derselben Charge n-Hexylbromid folgende Vergleichswerte gefunden worden:

| **Lösemittel** | **3-(1-Methyl-pentyl)thiophen** | **3-Hexylthiophen** | **Dithiophen** |
|---|---|---|---|
| Cyclopentyl-methylether | 0,5 | 95 | 0,0 |
| Cyclopentyl-methylether | 0,5 | 90,2 | 0,2 |
| Cyclopentyl-methylether | 0,6 | 86,5 | n.d. |
| 2-Methyltetra-hydrofuran | 1,1 | 81,7 | 3,1 |
| 2-Methyltetra-hydrofuran | 1,1 | 93,9 | n.d. |

Die Selektivität der Kupplung bezüglich der Bildung der Nebenkomponente 3-(1-Methylpentyl)thiophen ist bei dem hier vorgestellten Verfahren den bereits bekannten deutlich überlegen.

Zudem bietet Cyclopentylmethylether eine Reihe weiterer Vorteile gegenüber anderen Ethern. Hier sind als Beispiele der höhere Siedepunkt sowie das Mischungsverhalten mit Wasser, welches für metallorganische Reaktionen von großer Bedeutung ist, zu nennen:

| | **Cyclopentyl-methylether** | **2-Methyltetrahydrofuran** | **Tetrahydrofuran** | ***tert*-Butyl-methylether** | **Diethylether** |
|---|---|---|---|---|---|
| Siedepunkt [°C] | 106 | 78 | 65 | 55 | 35 |
| Schmelzpunkt [°C] | <-140 | -136 | -108,5 | -108,7 | -116,3 |
| Verdampfungs-enthalpie [kcal/kg] | 69,2 | 89,7 | 98,1 | 81,7 | 86,08 |
| Azeotrop mit Wasser, Siedepunkt [°C] | 83 | 71 | 64 | - | 34 |
| Löslichkeit in Wasser bei 23°C [gew-%] | 1,1 | 14 (20 °C) | unendlich | 4,8 | 6,5 |
| Löslichkeit von Wasser in Lösemittel bei 23°C [Gew.-%] | 0,3 | 4,4 (20 °C) | unendlich | 1,5 | 1,2 |

Auch die geringe Neigung zur Peroxidbildung zeichnet Cyclopentylmethylether als sicherer handhabbares Lösemittel für die Herstellung substituierter Thiophene aus.

Ein weiterer Gegenstand der Erfindung ist daher die Verwendung von Cycloalkylalkylether, insbesondere Cyclopentylmethylether, in der Kumada-Reaktion zur Herstellung substituierter Thiophene.

### Beispiele

### Beispiel 1 (vergleichend)

Zu 2,8 g Magnesiumspänen in 35 mL Tetrahydrofuran wurden 19,8 g Hexylbromid bei 50°C so zugetropft, dass ständig eine Exothermie zu erkennen war. Nach zweistündigem Refluxieren wurden 80 mg [1,3Bis-(Diphenylphosphino)-propan]-Nickel(II)-chlorid zugegeben. Anschließend wird bei 5°C 16,3 g 3-Bromthiophen zugetropft. Es wird 16 h bei 23°C gerührt, dann wird mit 60 mL Tetrahydrofuran verdünnt und auf 10 %ige (w/w) Salzsäure gegossen, die auf 0°C temperiert ist. Die organische Phase wird abgetrennt und die wässrige Phase dreimal mit 50 mL tert-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Aus der Rohlösung fällt ein Feststoff aus (Dithiophen), welcher abfiltriert wird. Es verbleiben 8,1 g Rohprodukt, aus dem nach längerem Stehen weiteres Dithiophen ausfällt. Die überstehende Lösung hat einen Gehalt von 73 %. Als Nebenkomponenten wurden identifiziert: 9 % Dodekan, 4 % 3-(1-Methylpentyl)thiophen.

### Beispiel 2 (vergleichend)

Zu 2,8 g Magnesiumspänen in 35 mL 2-Methyltetrahydrofuran wurden 19,8 g Hexylbromid bei 60-70°C so zugetropft, dass ständig eine Exothermie zu erkennen war. Nach zweistündigem Rühren bei 80-85°C wurden 80 mg [1,3Bis-(Diphenylphosphino)-propan]-Nickel(II)-chlorid zugegeben. Anschließend wird bei 15-20°C 16,3 g 3-Bromthiophen zugetropft. Es wird 3 h bei 23°C gerührt, dann wird der Ansatz auf 10 %ige Salzsäure (w/w) gegossen, die auf 0°C temperiert ist. Die organische Phase wird abgetrennt und die wässrige Phase dreimal mit 50 mL tert-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Das Rohprodukt hatte folgende Zusammensetzung: 11 % 3-Bromthiophen, 2 % Dodekan, 1,1 % 3-(1-Methylpentyl)thiophen, 82 % 3-Hexylthiophen, 3,1 % Dithiophen.

### Beispiel 3 (erfindungsgemäß)

Zu 2,8 g Magnesiumspänen in 35 mL Cyclopentylmethylether wurden 19,8 g Hexylbromid bei 90°C so zugetropft, dass ständig eine Exothermie zu erkennen war. Nach zweistündigem Refluxieren wurden 80 mg [1,3Bis-(Diphenylphosphino)-propan]-Nickel(II)-Chlorid zugegeben. Anschließend wird bei 10°C 16,3 g 3-Bromthiophen zugetropft. Es wird 2 h bei 23°C gerührt, dann wird 30 Minuten auf 100°C erhitzt und nach dem Abkühlen vorsichtig mit 10%iger Salzsäure (w/w) hydrolysiert, die auf 0°C temperiert ist. Die organische Phase wird abgetrennt und die wässrige Phase dreimal mit 50 mL *tert*-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Das Rohprodukt hatte folgende Zusammensetzung: 2,4 % 3-Bromthiophen, 7,4 % Dodekan, 0,6 % 3-(1-Methylpentyl)thiophen, 86,5 % 3-Hexylthiophen.

### Beispiel 4 (vergleichend)

Zu 2,8 g Magnesiumspänen in 35 mL 2-Methyltetrahydrofuran wurden 19,8 g Hexylbromid bei 60-70°C so zugetropft, dass ständig eine Exothermie zu erkennen war. Nach zweistündigem Refluxieren wurden 80 mg [1,3Bis-(Diphenylphosphino)-propan]-Nickel(II)-chlorid in 1 mL 2-Methyltetrahydrofuran zugegeben. Anschließend wird bei 20°C 16,3 g 3-Bromthiophen zugetropft. Es wird 16 h bei 23°C gerührt, dann wird vorsichtig mit 10 %iger Salzsäure (w/w) hydrolysiert, die auf 0°C temperiert ist. Die organische Phase wird abgetrennt und die wässrige Phase dreimal mit 50 mL *tert*-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Das Rohprodukt hatte folgende Zusammensetzung: 1,4 % 3-Bromthiophen, 1,7 % Dodekan, 1,1 % 3-(1-Methylpentyl)thiophen, 93,9 % 3-Hexylthiophen.

### Beispiel 5 (erfindungsgemäß)

Zu 17,5 g Magnesiumspänen in 215 mL Cyclopentylmethylether wurden 121,4 g Hexylbromid bei 80-85°C so zugetropft, dass ständig eine Exothermie zu erkennen war. Nach zweistündigem Rühren bei 80-85°C wurden 0,49 g [1,3Bis-(Diphenylphosphino)-propan]-Nickel(II)-chlorid in 8 mL Cyclopentylmethylether zugegeben. Anschließend wird bei 15°C 16,3 g 3-Bromthiophen zugetropft. Es wird 16 h bei 23°C gerührt, dann wird vorsichtig mit 10 %iger Salzsäure (w/w) hydrolysiert, die auf 0°C temperiert ist. Die organische Phase wird abgetrennt und die wässrige Phase dreimal mit 50 mL *tert*-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Das Rohprodukt hatte folgende Zusammensetzung: 0 % 3-Bromthiophen, 6,5 % Dodekan, 0,5 % 3-(1-Methylpentyl)thiophen, 88,2 % 3-Hexylthiophen.

### Beispiel 6 (vergleichend)

Zu 2,8 g Magnesiumspänen in 35 mL *tert*-Butylmethylether wurden 19,8 g Hexylbromid bei 60-70°C so zugetropft, dass ständig eine Exothermie zu erkennen war. Nach zweistündigem Refluxieren wurden 80 mg [1,3Bis-(Diphenylphosphino)-propan]-Nickel(II)-chlorid zugegeben. Anschließend wird bei 20°C 16,3 g 3-Bromthiophen zugetropft. Es wird 16 h bei 23°C gerührt und anschließend vorsichtig mit 10 %iger Salzsäure (w/w) hydrolysiert, die auf 0°C temperiert ist. Die organische Phase wird abgetrennt und die wässrige Phase dreimal mit 50 mL *tert*-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Das Rohprodukt hatte folgende Zusammensetzung: 10,3 % 3-Bromthiophen, 4,7 % Dodekan, 0,1 % 3-(1-Methylpentyl)thiophen, 77,4 % 3-Hexylthiophen, 5,2 % Dithiophen.

### Beispiel 7 (erfindungsgemäß)

Zu 52,3 g Magnesiumspänen in 645 mL Cyclopentylmethylether wurden 364,5 g Hexylbromid bei 72°C so zugetropft, dass ständig eine Exothermie zu erkennen war. Nach zweistündigem Rühren bei 80-85 °C wurden 1,47 g [1,3Bis-(Diphenylphosphino)-propan]-Nickel(II)-chlorid zugegeben. Anschließend wird bei 20°C 309,3 g 3-Bromthiophen zugetropft. Es wird 16 h bei 23°C gerührt, dann werden 260 mL Toluol zugegeben und anschließend vorsichtig mit 10 %iger Salzsäure (w/w) hydrolysiert, die auf 0°C temperiert ist. Die organische Phase wird abgetrennt und die wässrige Phase dreimal mit 250 mL Toluol extrahiert. Die vereinigten organischen Phasen werden mit je 250 mL Wasser und bei 23°C gesättigter Natriumchloridlösung gewaschen, über 20 g Magnesiumsulfat getrocknet, filtriert und eingeengt. Das Rohprodukt hatte folgende Zusammensetzung: 2,2 % 3-Bromthiophen, 4,1 % Dodekan, 0,5 % 3-(1-Methylpentyl)thiophen, 90,2 % 3-Hexylthiophen, 0,2 % Dithiophen.

### Beispiel 8 (erfindungsgemäß)

Zu 52,9 g Magnesiumspänen in 632 mL Cyclopentylmethylether wurden 364,5 g Hexylbromid bei 72°C so zugetropft, dass ständig eine Exothermie zu erkennen war. Nach zweistündigem Rühren bei 80-85 °C wurden 1,47 g [1,3Bis-(Diphenylphosphino)-propan]-Nickel(II)-chlorid zugegeben. Anschließend wird bei 20°C 309,3 g 3-Bromthiophen zugetropft. Es werden 260 mL Toluol zugegeben. Dann wird 16 h bei 23°C gerührt, dann wird vorsichtig mit 10 %iger Salzsäure (w/w) hydrolysiert, die auf 0°C temperiert ist. Die organische Phase wird abgetrennt, mit je 250 mL Wasser und bei 23°C gesättigter Natriumhydrogencarbonatlösung gewaschen und eingeengt. Das Rohprodukt hatte folgende Zusammensetzung: 0,8 % 3-Bromthiophen, 3,3 % Dodekan, 0,5 % 3-(1-Methylpentyt)thiophen, 95,2 % 3-Hexylthiophen, 0 % Dithiophen.

### Beispiel 9 (erfindungsgemäß)

Zu 2,8 g Magnesiumspänen in 35 mL Cyclopentylmethylether wurden 19,8 g Hexylbromid bei 70°C so zugetropft, dass ständig eine Exothermie zu erkennen war. Nach zweistündigem Rühren bei 80-85°C wurden bei 20°C 80 mg [1,3Bis-(Diphenylphosphino)-propan]-Nickel(II)-chlorid zugegeben. Anschließend wird bei 85°C 16,3 g 3-Bromthiophen zugetropft. Es wird eine Stunde zum Rückfluss erhitzt und anschließend 16 h bei 23°C gerührt. Der Ansatz wird mit Toluol verdünnt und auf 10 %ige Salzsäure (w/w) gegeben, die auf 0°C temperiert ist. Die organische Phase wird abgetrennt und die wässrige Phase dreimal mit 50 mL tert-Butylmethylether extrahiert. Die vereinigten organischen Phasen werden mit je 50 mL Wasser und bei 23°C gesättigter Natriumchloridlösung gewaschen, über 2,0 g Magnesiumsulfat getrocknet, filtriert und eingeengt. Das Rohprodukt hatte folgende Zusammensetzung: 6,1 % 3-Bromthiophen, 4,6 % Dodekan, 0,6 % 3-(1-Methylpentyl)thiophen, 84,9 % 3-Hexylthiophen, 2,5 % Dithiophen.

### Beispiel 10 (erfindungsgemäß)

Zu 52,3 g Magnesiumspänen in 678 mL Cyclopentylmethylether wurden 364,5 g Hexylbromid bei 72°C so zugetropft, dass ständig eine Exothermie zu erkennen war. Nach dreistündigem Rühren bei 80-85°C wurden 1,47 g [1,3Bis-(Diphenylphosphino)-propan]-Nickel(II)-chlorid zugegeben. Anschließend wird bei 20°C 309,3 g 3-Bromthiophen zugetropft. Es wird 16 h bei 23°C gerührt, dann werden 260 mL Toluol zugegeben und anschließend vorsichtig mit 10 %iger Salzsäure (w/w) hydrolysiert, die auf 0 C temperiert ist. Die organische Phase wird abgetrennt, mit je 250 mL Wasser und bei 23°C gesättigter Natriumhydrogencarbonatlösung gewaschen und eingeengt. Das Rohprodukt hatte folgende Zusammensetzung: 0 % 3-Bromthiophen, 6,1 % Dodekan, 0,6 % 3-(1-Methylpentyl)thiophen, 86,4 % 3-Hexylthiophen.

### Beispiel 11 (erfindungsgemäß)

Zu 52,3 g Magnesiumspänen in 678 mL Cyclopentylmethylether wurden 364,5 g Hexylbromid bei 72°C so zugetropft, dass ständig eine Exothermie zu erkennen war. Nach zweistündigem Rühren bei 80-85°C wurden 1,47 g [1,3Bis-(Diphenylphosphino)-propan]-Nickel(II)-chlorid zugegeben. Anschließend wird bei 20°C 309,3 g 3-Bromthiophen zugetropft. Es werden 260 mL Toluol zugegeben und 4 h bei 50°C und 12 h bei 23°C nachgerührt. Der Ansatz wird auf 10 %ige Salzsäure (w/w) gegeben, die auf 0°C temperiert ist. Die organische Phase wird abgetrennt, mit je 250 mL Wasser, bei 23°C gesättigter Natriumhydrogencarbonatlösung und erneut mit Wasser gewaschen und eingeengt. Das Rohprodukt hatte folgende Zusammensetzung: 4,6 % 3-Bromthiophen, 2,5 % Dodekan, 0,5% 3-(1-Methylpentyl)thiophen, 86,2 % 3-Hexylthiophen, 0,2 % Dithiophen.

### Beispiel 12 (erfindungsgemäß)

Zu 95,3 g Magnesiumspänen in 1098 mL Cyclopentylmethylether wurden 646,3 g Hexylbromid bei 70°C so zugetropft, dass ständig eine Exothermie zu erkennen war. Nach dreistündigem Rühren bei 80-85°C wurden 2,4 g [1,3Bis-(Diphenylphosphino)-propan]-Nickel(II)-chlorid zugegeben. Anschließend wird bei 20°C 491,1 g 3-Bromthiophen zugetropft. Es werden 415 mL Toluol zugegeben und 4 h bei 50°C und 12 h bei 23°C nachgerührt. Der Ansatz wird auf 10 %ige Salzsäure (w/w) gegeben, die auf 0°C temperiert ist. Die organische Phase wird abgetrennt, mit je 780 mL Wasser, bei 23°C gesättigter Natriumhydrogencarbonatlösung und erneut mit Wasser gewaschen und eingeengt. Das Rohprodukt hatte folgende Zusammensetzung: 1,3 % 3-Bromthiophen, 2,6 % Dodekan, 0,5 % 3-(1-Methylpentyl)thiophen, 90,6 % 3-Hexylthiophen, 0 % Dithiophen.

## Patentansprüche

1. Verfahren zur Herstellung von substituierten Thiophenen der allgemeinen Formel (I) 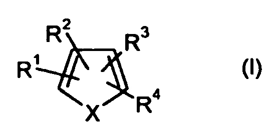 wobei
X Schwefel
und
R⁴ C₁-C₂₀-Alkyl,
R¹, R², R³ Wasserstoff, Halogen, C₁-C₂₀-Alkyl, C₅-C₆-Aryl oder Heteroaryl ist, durch Umsetzung
A) eines halogenierten Heteroaromaten der allgemeinen Formel (II) 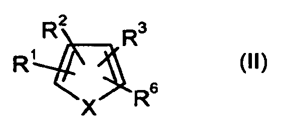 wobei
X die für Formel (I) angegebene Bedeutung und
R⁶ Brom, Iod oder Chlor ist und
R¹, R² und R³ die für Formel (I) angegebene Bedeutung haben,
mit einem Grignard-Reagenz der allgemeinen Formel (III)
R⁴MgHal (III)
wobei
R⁴ die für Formel (I) angegebene Bedeutung hat und
Hal Brom, Iod oder Chlor ist oder
B) Umsetzung des halogenierten Heteroaromaten der Formel (II) mit Magnesium zunächst zu einer Grignard-Verbindung der allgemeinen Formel (IIIa) 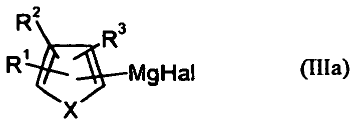 wobei
Hal Brom, Iod oder Chlor ist und
X und R¹, R² und R³ die für Formel (I) angegebene Bedeutung haben,
und weiterer Umsetzung mit einer halogenierten Verbindung der allgemeinen Formel (IV)
R⁴Hal (IV)
wobei
R⁴ die für Formel (I) angegebene Bedeutung hat und
Hal Brom, Iod oder Chlor ist,
wobei die Umsetzungen A) oder B) jeweils in Gegenwart eines Ni- oder Pd-Katalysators durchgeführt werden, **dadurch gekennzeichnet, dass** das Verfahren in Gegenwart von Cycloalkylalkylether als Lösungsmittel und gegebenenfalls einem oder mehreren weiteren Lösungsmitteln durchgeführt wird.

2. Verfahren nach Anspruch 1, **dadurch gekennzeichnet, dass** der Cycloalkylalkylether Cyclopentylmethylether ist.

3. Verfahren nach Anspruch 1, **dadurch gekennzeichnet, dass** der Cycloalkylalkylether Cyclopentylmethylether und das weitere Lösungsmittel ein Lösungsmittel aus der Gruppe Toluol, Tetrahydrofuran, Methyltetrahydrofuran, tert.-Butylmethylether, Diethylether oder deren Mischungen ist.

4. Verfahren nach einem der vorherigen Ansprüche, **dadurch gekennzeichnet, dass** es bei einer Temperatur zwischen -30 und 106°C durchgeführt wird.

5. Verfahren nach einem der vorherigen Ansprüche, **dadurch gekennzeichnet, dass** der Katalysator [1,3-Bis-(Diphenylphosphino)-propan]-Nickel(II)-chlorid ist.

6. Verfahren nach einem der vorherigen Ansprüche, **dadurch gekennzeichnet, dass** X in Formel (I), (II)und (IIIa) Schwefel ist.

7. Verfahren nach einem der vorherigen Ansprüche 1 bis 6, **dadurch gekennzeichnet, dass** R⁴ ein C₆-C₁₀- oder C₁₂-Alkylrest ist.

8. Verwendung von Cycloalkylalkylether als Lösungsmittel in der Kumada-Reaktion zur Herstellung substituierter Thiophene.

9. Verwendung nach Anspruch 8, **dadurch gekennzeichnet, dass** der Cycloalkylalkylether Cyclopentylmethylether ist.

## Claims

1. Process for the preparation of substituted thiophenes of the general formula (I) 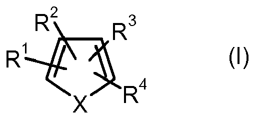 where
X is sulphur
and
R⁴ is C₁-C₂₀-alkyl,
R¹, R², R³ is hydrogen, halogen, C₁-C₂₀-alkyl, C₅-C₆-aryl or heteroaryl, by reaction
A) of a halogenated heteroaromatic of the general formula (II) 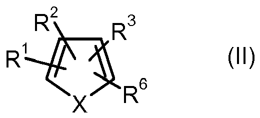 where
X has the meaning given for formula (I) and
R⁶ is bromine, iodine or chlorine and
R¹, R² and R³ have the meaning given for formula (I),
with a Grignard reagent of the general formula (III)
R⁴MgHal (III)
where
R⁴ has the meaning given for formula (I) and
Hal is bromine, iodine or chlorine or
B) reaction of the halogenated heteroaromatics of the formula (II) with magnesium firstly to give a Grignard compound of the general formula (IIIa) 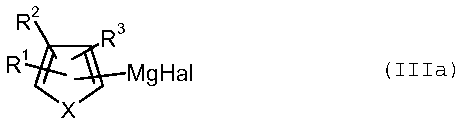 where
Hal is bromine, iodine or chlorine and
X and R¹, R² and R³ have the meaning given for formula (I),
and further reaction with a halogenated compound of the general formula (IV)
R⁴Hal (IV)
where
R⁴ has the meaning given for formula (I) and
Hal is bromine, iodine or chlorine,
where the reactions A) or B) are in each case carried out in the presence of an Ni or Pd catalyst, **characterized in that** the process is carried out in the presence of cycloalkyl alkyl ether as solvent and optionally one or more further solvents.

2. Process according to Claim 1, **characterized in that** the cycloalkyl alkyl ether is cyclopentyl methyl ether.

3. Process according to Claim 1, **characterized in that** the cycloalkyl alkyl ether is cyclopentyl methyl ether and the further solvent is a solvent from the group consisting of toluene, tetrahydrofuran, methyltetrahydrofuran, tert-butyl methyl ether, diethyl ether or mixtures thereof.

4. Process according to one of the preceding claims, **characterized in that** it is carried out at a temperature between -30 and 106°C.

5. Process according to one of the preceding claims, **characterized in that** the catalyst is [1,3-bis(diphenylphosphino)propane]nickel(II) chloride.

6. Process according to one of the preceding claim, **characterized in that** X in formula (I), (II) and (IIIa) is sulphur.

7. Process according to one of the preceding claims 1 to 6, **characterized in that** R⁴ is a C₆-C₁₀- or C₁₂-alkyl radical.

8. Use of cycloalkyl alkyl ether as solvent in the Kumada reaction for the preparation of substituted thiophenes.

9. Use according to Claim 8, **characterized in that** the cycloalkyl alkyl ether is cyclopentyl methyl ether.

## Revendications

1. Procédé de fabrication de thiophènes substitués de formule générale (I) 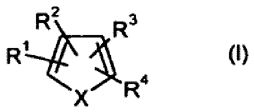 dans laquelle
X représente le soufre
et
R⁴ représente un alkyle en C₁-C₂₀,
R¹, R², R³ représentent l'hydrogène, un halogène, un alkyle en C₁-C₂₀, un aryle en C₅-C₆ ou un hétéroaryle, par mise en réaction de
A) un composé hétéroaromatique halogéné de formule générale (II) 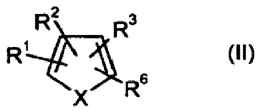 dans laquelle
X a la signification donnée pour la formule (I) et
R⁶ représente le brome, l'iode ou le chlore, et
R¹, R² et R³ ont la signification donnée pour la formule (I)
avec un réactif de Grignard de formule générale (III)
R⁴MgHal (III)
dans laquelle
R⁴ a la signification donnée pour la formule (I) et
Hal représente le brome, l'iode ou le chlore, ou
B) mise en réaction du composé hétéroaromatique halogéné de formule (II) tout d'abord avec du magnésium pour former un composé de Grignard de formule
générale (IIIa) 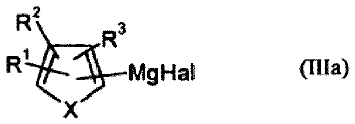 dans laquelle
Hal représente le brome, l'iode ou le chlore, et
X et R¹, R² et R³ ont la signification donnée pour la formule (I),
et mise en réaction ultérieure avec un composé halogéné de formule générale (IV)
R⁴Hal (IV)
dans laquelle
R⁴ a la signification donnée pour la formule (I) et
Hal représente le brome, l'iode ou le chlore,
les réactions A) ou B) étant chacune réalisées en présence d'un catalyseur de Ni ou Pd, **caractérisé en ce que** le procédé est réalisé en présence d'un éther de cycloalkyle et d'alkyle en tant que solvant et éventuellement d'un ou de plusieurs solvants supplémentaires.

2. Procédé selon la revendication 1, **caractérisé en ce que** l'éther de cycloalkyle et d'alkyle est l'éther de cyclopentyle et de méthyle.

3. Procédé selon la revendication 1, **caractérisé en ce que** l'éther de cycloalkyle et d'alkyle est l'éther de cyclopentyle et de méthyle et le solvant supplémentaire est un solvant du groupe constitué par le toluène, le tétrahydrofurane, le méthyltétrahydrofurane, l'éther de tert.-butyle et de méthyle, l'éther diéthylique ou leurs mélanges.

4. Procédé selon l'une quelconque des revendications précédentes, **caractérisé en ce qu'**il est réalisé à une température comprise entre -30 et 106 °C.

5. Procédé selon l'une quelconque des revendications précédentes, **caractérisé en ce que** le catalyseur est le chlorure de [1,3-bis-(diphénylphosphino)-propane]-nickel (II).

6. Procédé selon l'une quelconque des revendications précédentes, **caractérisé en ce que** X dans la formule (I), (II) et (IIIa) est le soufre.

7. Procédé selon l'une quelconque des revendications 1 à 6, **caractérisé en ce que** R⁴ est un radical alkyle en C₆-C₁₀ ou en C₁₂.

8. Utilisation d'un éther de cycloalkyle et d'alkyle en tant que solvant dans la réaction de Kumada pour la fabrication de thiophènes substitués.

9. Utilisation selon la revendication 8, **caractérisée en ce que** l'éther de cycloalkyle et d'alkyle est l'éther de cyclopentyle et de méthyle.
